# EUROPEAN PATENT APPLICATION

(11) **EP 1 705 445 A1**
(43) Date of publication of application: **27.09.2006**
(21) Application number: 05004763.8
(22) Date of filing: 04.03.2005
(51) Int. Cl.: F28D 1/03, B23K 26/02

(54) **Method for the production of plate type heat exchangers and related apparatus**

(71) Applicant: METHANOL CASALE S.A., 6900 Lugano-Besso (CH)
(72) Inventor: Filippi, Ermanno, CH-6976 Castagnola (CH); Rizzi, Enrico, 22070 Grandate (CO) (IT); Tarozzo, Mirco, CH-6853 Ligornetto (CH)
(74) Representative: Zardi, Marco

(57) **Abstract**

A method for the production of plate type heat exchangers (10) comprising the operative steps of:
-juxtaposing a couple of metal plates (12, 14),
-welding each other said juxtaposed plates (12, 14) at least perimetrically,
-defining an inner chamber (16) between said metal plates (12, 14), intended to be crossed by an operating heat exchange fluid,

said operative welding step of the juxtaposed plates (12, 14) being carried out by supplying heat directly to each of said metal plates (12, 14).

## Description

### Field of application

The present invention refers, in its most general aspect, to a method for the production of so-called plate type heat exchangers, in other words heat exchangers consisting of box-shaped, parallelepiped, flattened bodies formed from two juxtaposed rectangular plates, joined each other in an appropriate spaced relationship, so as to define an inner chamber, intended to be crossed by an operating heat exchange fluid.

Heat exchangers of the aforementioned type have advantageous use in the formation of heat exchange units of chemical reactors, where they are necessary for optimal completion of exothermal or endothermic chemical reactions: for example, in synthesis reactions of ammonia, methanol, formaldehyde or, respectively, styrene, to remove or, respectively, to provide heat from/to a reaction environment, generally a catalytic bed, so as to control its temperature within a narrow range around a precalculated theoretical value.

In particular the invention refers to a method for the production of plate type heat exchangers comprising the operating steps of:
- juxtaposing a couple of metal plates,
- welding each other at least perimetrically said juxtaposed plates,
- defining an inner chamber between said metal plates, intended to be crossed by an operating heat exchange fluid.

The invention also refers to an apparatus for carrying out the aforementioned method for the production of plate type heat exchangers of the type considered, as well as to the heat exchangers obtained with said method.

### Prior Art

In the field of chemical reactors, and in many other industrial applications, it is well known the use of heat exchange units comprising a plurality of heat exchangers and, in particular, so-called plate type heat exchangers.

In order to produce such plate type heat exchangers, it is necessary to weld each other a couple of juxtaposed metal plates along a predetermined perimeter; in some cases, in order to rigidify the exchangers, in addition to the perimetric welding, spot weldings is also carried out that joins each other the metal plates in areas that are situated within the perimeter of the plates themselves, areas that are preferably distributed regularly along the planar surface of said metal plates.

Thereafter, an inner chamber is defined between said metal plates, intended to be crossed by an operating heat exchange fluid.

The inner chamber is formed through a so-called swelling step, i.e. a step of introduction of a pressurized fluid between said plates that are, in this case, welded to form a seal against pressurized fluid. Generally, for swelling pressures below about 40-50 bar one operates pneumatically, whereas for greater pressure one operates hydraulically.

Moreover, the inner chamber is in fluid communication with the outside of the exchanger through an inlet opening and an outlet opening of said operating fluid. Such openings are generally made after the swelling step, having carried out a perimetric welding along the entire perimeter of the metal plates.

The pressurized fluid is injected through a small opening that, possibly, suitably enlarged and equipped with a suitable gate, can transform into the aforementioned inlet opening or outlet opening.

Furthermore, a path is generally defined in the inner chamber to be crossed by the operating heat exchange fluid.

Generally, for such a production, automated welding apparatuses are used that carry out autogenous type welding, in other words obtaining a union between the two plates, by fusion of the metal of which they consist, without using filler material.

According to a constant teaching of the prior art, the weldings are formed through the supply of heat in a direct manner to one of said metal plates.

A widely adopted apparatus comprises a head carrying a laser beam source. The welding takes place through laser beams that are orientated on the outer surface of the aforementioned plate: in this way areas of the couple of metal plates heat up by conduction, until they are melted in a predetermined contact area of the couple of metal plates.

Although advantageous from various points of view, the method for the production of plate type heat exchangers according to what has been schematically described above has recognized drawbacks.

The main one is that one is faced with a progressive deformation and/or distortion of the plates during the welding operations, caused by the shrinkage tensions that occur during the cooling of the weldings themselves, i.e. during the cooling of the heated metal areas.

Such a phenomenon, which is always present, is all the more evident the greater the size (length and/or width) of the heat exchanger to be made and the greater the thickness of the juxtaposed plates to be welded. In the case of plate type heat exchangers for the inside of chemical reactors, for example, considerable sizes and, frequently, thicknesses close to the technological limits for laser welding are required; this is the case, for example, of reactors for ammonia synthesis, in which one is forced to choose large thicknesses (for example, 2 - 2.5 mm) due to the aggressiveness of the process fluid (nitriding phenomenon), or of reactors for methanol synthesis that have plate type heat exchangers subjected to high differential pressures between the inside and outside of the plate type exchangers themselves, since the pressure of the cooling fluid circulating inside the plate type exchangers is different to the pressure of the process fluid.

Basically, the progressive deformation and/or distortion of the plate type heat exchangers can, for long plates, also lead to shifts in planarity of the order of one hundred millimetres.

Such a situation makes it very difficult, if not at times impossible, to assemble a plurality of plate type heat exchangers to constitute a heat exchange unit in the reactor, and in any case leads to a disuniformity in the spacing of the plate type heat exchangers, with negative consequences at the process level (for example, in terms of performances of the reactor, in terms of generation of hot points inside the catalytic mass provided in the reactor, etc.).

For these reasons, it is necessary to provide further operations that take the planarity of the plate type heat exchangers below a predetermined value, before they are mounted at work.

Generally, one operates by applying some constraints to the plate type heat exchanger during the introduction step of the pressurized fluid between the plates of the exchanger itself, to counteract the tensions that generate during the welding. Such operations are quite complex and undoubtedly involve a significant slowing in the production of the plate type heat exchangers.

### Summary of the invention

The technical problem underlying the present invention is that of devising and providing a method for the production of plate type heat exchangers capable of overcoming the drawbacks quoted with reference to the prior art, in a simple and effective manner.

According to the present invention, based upon the experiments carried out by the Applicant in sharp contrast with the constant teaching of the prior art, this problem is solved by a method for the production of plate type heat exchangers, of the aforementioned type, characterized in that said operative welding step of the juxtaposed plates is carried out by supplying heat directly to each of said metal plates.

Further characteristics and advantages of the method for the production of heat exchangers according to the present invention shall become clear from the following description of a preferred embodiment thereof, given for indicating and not limiting purposes with reference to the attached drawings.

### Brief description of the drawings

Figure 1 schematically represents a perspective cutaway view of a plate type heat exchanger, obtained with the method according to the invention.
Figure 2 schematically represents a perspective view of a couple of metal plates subjected to an operative welding step according to the method of the invention.
Figure 3 schematically represents an enlarged cross section view of a portion of the couple of metal plates of figure 2, according to a first embodiment of the method.
Figure 4 schematically represents an enlarged cross section view of a portion of the couple of metal plates of figure 2, according to a further embodiment of the method.

### Detailed description of a preferred embodiment

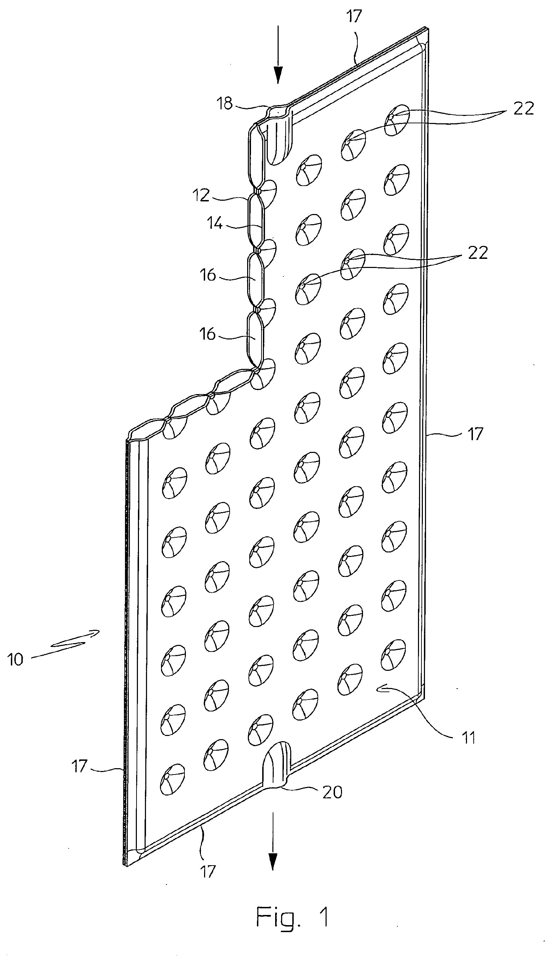

With reference to figure 1, a plate type heat exchanger in accordance with the present invention is shown globally indicated with 10.

The heat exchanger 10 consists of a box-shaped, parallelepiped, flattened body 11.

The exchanger 10 is produced from two rectangular metal plates, upper 12 and lower 14, preferably of the same thickness.

The method comprises the operative steps of:
- juxtaposing said couple of metal plates 12 and 14,
- welding each other said juxtaposed plates 12, 14 at least perimetrically,
- defining an inner chamber 16 between said metal plates 12, 14, intended to be crossed by an operating heat exchange fluid.

Of course, the individual plates 12, 14 can be obtained by placing two or more small sized plates (not represented) next to each other and joining them (for example through welding).

The definition of such an inner chamber 16 can be obtained by welding each other the plates to form a seal against pressurized fluid, and introducing a pressurized fluid between said plates.

The weldings between the two plates 12 and 14 are perimetric weldings 17, carried out along at least part of the perimeter of such plates 12 and 14.

In practice, at the end of the production, the two plates 12 and 14 are perimetrically joined each other and are in an appropriate spaced apart relationship, so as to define said inner chamber 16.

This chamber 16 is in fluid communication with the outside of the exchanger through an inlet opening 18 and an outlet opening 20 of said operating fluid.

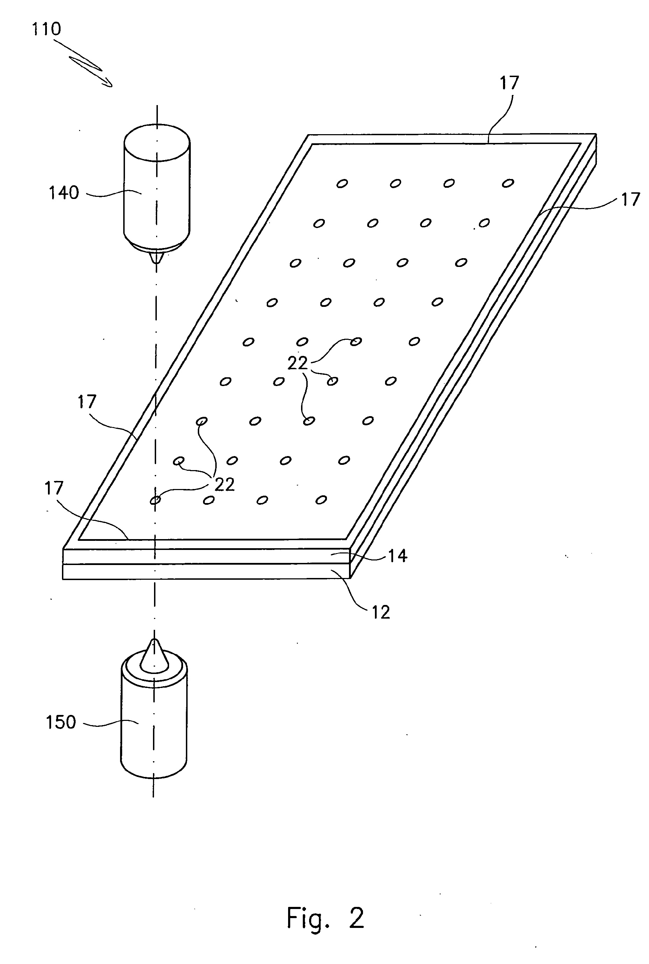

In some cases, like in the example of figure 2, as well as the perimetric welding 17, before the operative introduction step of the pressurized fluid, spot weldings 22 are also made that join each other the metal plates 12 and 14 also in areas that are within the perimeter of the plates 12 and 14 themselves, areas that are preferably regularly distributed along the planar surface of said metal plates 12 and 14.

In accordance with an aspect of the present invention, said operative welding step is carried out by supplying heat directly to each of said metal plates 12 and 14. Said supply of heat in direct manner is carried out through at least one heat source that is faced with each of said juxtaposed metal plates 12 and 14.

In the example of figure 2, two heat sources are provided, each facing a metal plate.

An apparatus 110 for carrying out the aforementioned method is thus partially shown in figure 2. The apparatus 110 comprises a substantially flat support structure (preferably arranged horizontally) for said couple of juxtaposed metal plates 12 and 14, per se conventional and therefore not illustrated, moving means, also per se conventional and therefore not illustrated, to move the couple of metal plates 12 and 14 below a head 140 carrying a heat source, such as a laser beam source, arranged in a predetermined position. Such moving means can, for example, comprise seaming clamps for said plates 12 and 14, said clamps being able to slide on guides.

Alternatively, a plurality of said heads 140 carrying heat sources can be provided.

In accordance with an aspect of the present invention, such an apparatus comprises a further head 150 carrying a heat source, such as a laser beam source, below the support structure, facing the other metal plate, arranged in a predetermined position, providing a through opening facing said further head 150 in said support structure. Alternatively, a plurality of said further heads 150 can be provided, also facing more than one through opening.

The operation of the apparatus according to the invention is specified hereafter.

The couple of metal plates 12 and 14 is positioned on the support structure of the apparatus, one juxtaposed on the other.

Through the moving means, the couple of plates 12 and 14 is moved on said support structure, in such a way that the laser beams emitted by the source with which the head 140 is equipped strike the upper plate 12 to make perimetric weldings 17 along the perimeter of the plates 12 and 14 and possibly spot weldings 22.

According to the invention, through the moving means, the couple of plates 12 and 14 is moved on said support structure so that the laser beams emitted by the further head 150, crossing the through opening of said support structure, strike the lower plate 14 to make perimetric weldings 17 along the perimeter of the plates 12 and 14 and possibly spot weldings 22.

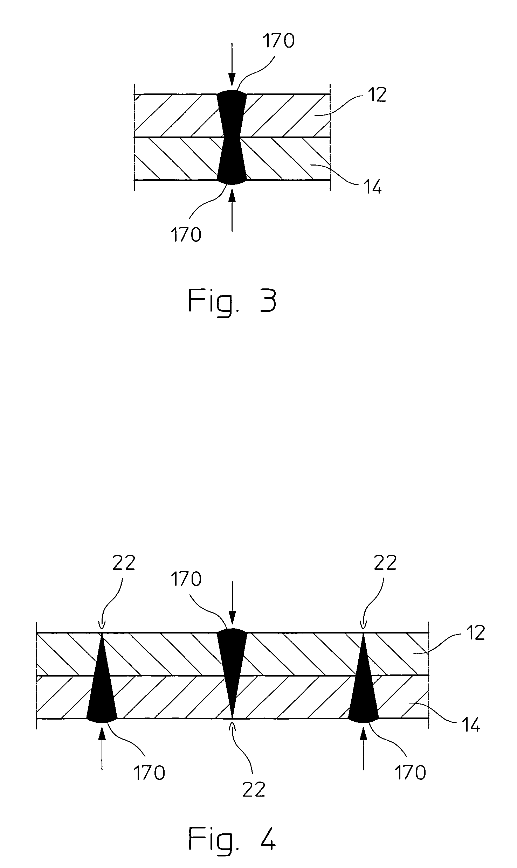

More specifically and preferably, as schematized in figure 3, the two laser beams coming respectively from said head 140 and from said further head 150 strike areas of the heat exchanger 10 that are opposite each other, so that the heat is supplied in a substantially equal manner on the two parts of the welding to be carried out (in the same way, if the heads 140 are a plurality thereof, a plurality of further heads 150 is placed opposite them).

By doing so, an optimal planarity of the exchanger 10 produced is obtained. In particular, figure 3 shows that the weldings according to the invention have symmetrical welding beads 170, by welding beads 170 meaning the areas of metal material that, during the welding, have reached the melting temperature.

Alternatively, as shown in figure 4, the perimetric welding 17 and the possible spot weldings 22 are each obtained by alternating the use of the heads 140 and 150: by doing so, it has been noted that a compensation of the welding distortions is obtained, which allows the planarity of the exchanger 10 to be maintained within predetermined values. In particular, figure 4 shows that the weldings according to the invention have welding beads 170 alternating with each other.

Basically, the perimetric welding 17 is formed by associating welding portions that are respectively produced by the head 140 and by the further head 150: the welding portions produced by the head 140 are contiguous to those produced by the further head 150, i.e., in other words, there is an alternation between the portions produced by the head 140 and those produced by the further head 150. Preferably, to ensure that the perimetric welding is continuous 17 and therefore forms a seal against pressurized fluid, the weldings of the ends of the welding portions overlap the respective ends of the contiguous welding portions.

In the same way, as far as the spot weldings 22 are concerned, the weldings generated by the head 140 are alternated with the spot weldings 22 generated by the further head 150.

It should be specified that, in the present invention, the term alternate is meant in its widest sense, that is, for example in the case of a row of spot weldings, a welding carried out from the head and a welding carried out from the further head can follow one another, or two weldings carried out from the head and two weldings carried out from the further head can follow one another, and so on.

Basically, the plate type heat exchangers obtained with the aforementioned method are characterized in that they have weldings with balanced shrinkage tensions, both in the case of symmetrical welding beads (figure 3), and in the case of alternating welding beads (figure 4).

It should be noted that the described apparatus 110 provides welding heads 140 and 150 that are in a predetermined position with respect to the support structure, the moving means moving (i.e., generally, advancing) the couple of metal plates 12 and 14 on said support structure.

Alternatively, moving means for the welding heads 140 and 150 can be provided, which are thus mobile with respect to the support structure, the couple of metal plates 12 and 14 being arranged in a predetermined position on said support structure.

From the previous description it can clearly be seen that the method for the production of plate type heat exchangers according to the invention solves the technical problem and achieves numerous advantages the first of which lies in the fact that exchangers with an unusual planarity are obtained, also in the case of exchangers of great thickness and/or of great length and/or width.

Another advantage is that the exchangers obtained can easily be installed, for example in chemical reactors.

Another advantage is that the exchangers obtained ensure optimal performances of the reactor, thanks to the uniformity in the spacing between the plate type exchangers that derives from their optimal planarity, to the great advantage of the heat exchange efficiency.

A further advantage is that exchangers are obtained that are, structurally, particularly strong, since the tensions that are generated in the welding according to the invention are well balanced.

Of course, a man skilled in the art can bring numerous modifications and variants to the method for the production of plate type heat exchangers described above, in order to satisfy specific and contingent requirements, all of which are covered by the scope of protection of the present invention, as defined by the following claims.

## Claims

1. Method for the production of plate type heat exchangers (10) comprising the operative steps of:
- juxtaposing a couple of metal plates (12, 14),
- welding each other said juxtaposed plates (12, 14) at least perimetrically,
- defining an inner chamber (16) between said metal plates (12, 14), intended to be crossed by an operating heat exchange fluid,
**characterized in that** said operative welding step of the juxtaposed plates (12, 14) is carried out by supplying heat directly to each of said metal plates (12, 14).

2. Method according to claim 1, **characterized in that** said welding between said plates (12, 14) is carried out along at least part of the perimeter of said plates (12, 14).

3. Method according to claim 1, **characterized in that** said welding between said plates (12, 14) also takes the form of spot weldings (22) that join each other the metal plates (12, 14) in areas situated within the perimeter of the plates (12, 14).

4. Method according to claim 1, **characterized, in that** said supply of heat in direct manner is carried out through at least one heat source (140, 150) that faces each of said juxtaposed metal plates (12, 14).

5. Method according to claim 4, **characterized in that** said welding is obtained by arranging said at least one heat sources (140, 150), respectively facing the two metal plates (12, 14), opposite each other, so that the heat is supplied in a substantially equal manner on the two sides of the welding to be carried out.

6. Method according to claim 1, **characterized in that** said supply of heat in direct manner is carried out using a laser beam source.

7. Apparatus (110) for the production of plate type heat exchangers (10), of the type comprising a substantially flat support structure for a couple of metal plates (12, 14), juxtaposed each other, moving means to move said couple of metal plates (12, 14) below at least one head (140) carrying a heat source, arranged in a predetermined position facing one of said metal plates, **characterized in that** it comprises at least one further head (150) carrying a heat source facing the other metal plate, providing in said support structure at least one through opening facing said at least one further head (150).

8. Plate type heat exchanger (10), consisting of a box-shaped, parallelepiped, flattened body (11) formed from two plates (12, 14) joined each other and that are in an appropriate spaced relationship, so as to define an inner chamber in fluid communication with the outside of the exchanger through an inlet opening (18) and an outlet opening (20) of an operating heat exchange fluid, **characterized in that** it has weldings with balanced shrinkage tensions.
